# EUROPEAN PATENT APPLICATION

(11) **EP 1 787 594 A2**
(43) Date of publication of application: **23.05.2007**
(21) Application number: 06124501.5
(22) Date of filing: 21.11.2006
(51) Int. Cl.: A61B 18/04, A61B 19/00

(54) **System and method for improved ablation of tumors**

(30) Priority: 22.11.2005 US 286542
(71) Applicant: General Electric Company, Schenectady, NY 12345 (US)
(72) Inventor: Mahesh, Prakash, Hoffman Estates, IL 60192 (US); Morita, Mark M., Arlington Heights, IL 60005 (US)
(74) Representative: Illingworth-Law, William Illingworth

(57) **Abstract**

Certain embodiments of the present invention provide methods (100) and systems for improved tumor ablation. Certain embodiments include determining a distance between a tumor (320) and at least one of a plurality of landmarks (330, 335) bounding the tumor (320) in at least one acquired image (310) of an area of interest for a patient (130), obtaining positional data for an ablation instrument (340) (140), and displaying a position of the ablation instrument (340) with respect to the tumor (320). Additionally, a position of the ablation instrument (340) may be dynamically displayed on the fluoroscopic image (350) and/or the at least one acquired image (310) during tumor ablation. Furthermore, a location cursor on the fluoroscopic image (350) may be linked with a location cursor on the one or more acquired images (310) (150). In certain embodiments, a location of a tip of the ablation instrument (340) may be dynamically displayed with respect to a starting location and an ending location of the tumor (320).

## Description

### FIELD OF THE INVENTION

The present invention generally relates to tumor ablation. In particular, the present invention relates to systems and methods for improved ablation of tumors using a PACS.

### BACKGROUND OF THE INVENTION

Medical imaging systems may be used to capture images to assist a physician in making an accurate diagnosis. For example, a physician may use one or more images to visually identify a tumor, lesion, and/or other anomalous structure in a patient. As another example, a physician may compare images taken over a series of patient visits to examine the evolution of a structure and/or to evaluate the effectiveness of a treatment. That is, the physician may examine morphological changes, such as changes in size and/or shape, of a tumor to evaluate its characteristics and/or the effectiveness of therapy.

Image data may come from a variety of sources. Images may be generated and/or acquired from one or more imaging sessions and involve different modalities (e.g., ultrasound (US), magnetic resonance (MR), computed tomography (CT), x-ray, positron emission tomography (PET), nuclear, thermal, optical, video, etc.), views, slices, and/or protocols. Images may originate from a single source or be a result of calculation (e.g., fused or compound images from multiple modalities).

An image processing system may combine image exposures with reference data to construct a three-dimensional (3D) volumetric data set. The 3D volumetric data set may be used to generate images, such as slices, or a region of interest from the object. For example, the image processing system may produce from the volumetric data sets sagittal, coronal, and/or axial views of a patient's spine, knee, or other area.

PET scanning can be used to generate images representing metabolic activity in, for example, a patient. A radioactive tracer, such as, Fluorine-18 2-fluoro-2-deoxy-D-glucose (FDG), may be injected into a patient. FDG mimics glucose and, thus, may be taken up and retained by tissues that require glucose for their activities. Tissues with higher metabolic activity will contain more of the tracer. A PET scanner allows detection of the tracer through its radioactive decay. Thus, by detecting and determining the location of the tracer, a PET scanner may be used to generate images representing metabolic activity.

The resolution of PET data may not be particularly high as compared to other imaging technologies, such as, for example, CT. For example, a voxel in PET data may be 4mm per axis. This low resolution makes it difficult to precisely define the location and contours of the detected structures. PET data may be fused with CT data, for example, to aid in locating and evaluating the detected active tumors.

Tumors may be treated in a variety of ways. For example, tumors may be irradiated, chemically treated, and/or excised. Currently, interventional radiologists performing ablation of tumors have multiple ways to perform the procedure: using ultrasound, using needle electrodes, etc. For example, needle electrodes may be used to heat or cook a tumor with high temperatures for a certain period of time. Currently, tumor ablation is a trial and error procedure. That is, an interventional radiologist looks at CT images both with and without dye contrasts, plan the ablation procedure and perform the tumor ablation. After the procedure, a PET scan may be taken to ensure that the tissues and cells are dead in the tumorous area.

Additionally, current ablation methods approximate or guess regarding the 3D location of a tumor. A radiologist may look at CT studies and realtime two-dimensional (2D) fluoroscopic images and navigate to the location of the tumor. Since the realtime image is 2D, the radiologist must currently make some judgment regarding the location of the tumor in the z-axis (coronal plane). In many cases, tumor ablation is performed, and a post-procedure PET scan is reviewed in order to re-do the procedure to cook more tissues. Such imprecise repetition is painful and suboptimal both for the radiologist and the patient.

Thus, there is a need for improved tumor ablation. There is a need for systems and methods for better location of a tumor in a patient. There is a need for systems and methods linking image data to positional data for improved tumor ablation.

### BRIEF SUMMARY OF THE INVENTION

Certain embodiments of the present invention provide methods and systems for improved tumor ablation. Certain embodiments of a method include determining a distance between a tumor and at least one of a plurality of landmarks bounding the tumor in at least one acquired image of an area of interest for a patient, obtaining positional data for an ablation instrument, and displaying a position of the ablation instrument with respect to the tumor.

Certain embodiments of the method may also include registering the at least one acquired image with a fluoroscopic image. Additionally, the method may include dynamically displaying a position of the ablation instrument on at least one of the fluoroscopic image and the at least one acquired image during tumor ablation. Furthermore, the method may include linking a location cursor on the fluoroscopic image with a location cursor on the one or more acquired images.

In certain embodiments, the method may include dynamically displaying a location of a tip of the ablation instrument with respect to a starting location and an ending location of the tumor. The method may include determining distances between the tumor and the plurality of landmarks to identify a location of the tumor in the at least one acquired image. In certain embodiments, the distance between the tumor and one or more landmarks may be marked on one or more of the acquired images. In certain embodiments, the method may further include determining a depth of the ablation instrument in the patient. In certain embodiments, the one or more acquired images may include a plurality of images obtained with contrast dye and without contrast dye.

Certain embodiments provide a tumor ablation system including a processing unit receiving positional data from an ablation instrument and a display unit displaying a dynamically-updating image of an area of interest and at least one image slice depicting the area of interest. Each of the at least one image slices includes a depiction of a tumor and a plurality of landmarks surrounding the tumor. The display unit displays a position of the ablation instrument concurrently on the dynamically-updating image and on the at least one image slice.

In certain embodiments, the ablation instrument comprises a needle electrode, for example. In certain embodiments, the display unit displays the position of the ablation device and a position of the tumor in a three-dimensional coordinate system. In certain embodiments, the display unit facilitates indicating a distance between at least one of the plurality of landmarks and the tumor on at least one of the dynamically-updating image and the at least one image slice. The display unit may be a picture archiving and communication system workstation or other display workstation or terminal, for example. In certain embodiments, the processing unit registers the dynamically-updating image and the at least one image slice. In certain embodiments, the display unit dynamically displays a tip of the ablation instrument with respect to a starting location and an ending location of the tumor.

Certain embodiments provide a computer-readable medium including a set of instructions for execution on a computer or other processor. The set of instructions may include a distance routine configured to determine at least one distance between a tumor and a plurality of landmarks identified in at least one image slice of a patient area, a tracking routine capable of obtaining positional data for an ablation instrument, and a display routine for displaying three-dimensional position information for the ablation instrument with respect to the tumor based on the positional data.

In certain embodiments, the display routine displays a cursor location indicative of the ablation instrument concurrently on the at least one image slice of a patient area and a fluoroscopic image of the patient area. In certain embodiments, the display routine dynamically displays a location of a tip of the ablation instrument with respect to a starting location and an ending location of the tumor. In certain embodiments, the display routine indicates the at least one distance between the tumor and the plurality of landmarks on a dynamically-updating image displayed during tumor ablation.

### BRIEF DESCRIPTION OF SEVERAL VIEWS OF THE DRAWINGS

FIG. 1 illustrates a flow diagram for a method 100 for tumor ablation in accordance with an embodiment of the present invention.
FIG. 2 illustrates an exemplary Picture Archiving and Communication System (PACS) used in accordance with an embodiment of the present invention.
FIG. 3 illustrates exemplary images showing tumor to landmark distance and need location in accordance with an embodiment of the present invention.

The foregoing summary, as well as the following detailed description of certain embodiments of the present invention, provided by way of example, will be better understood when read in conjunction with the appended drawings. For the purpose of illustrating the invention, certain embodiments are shown in the drawings. It should be understood, however, that the present invention is not limited to the arrangements and instrumentality shown in the attached drawings.

### DETAILED DESCRIPTION OF THE INVENTION

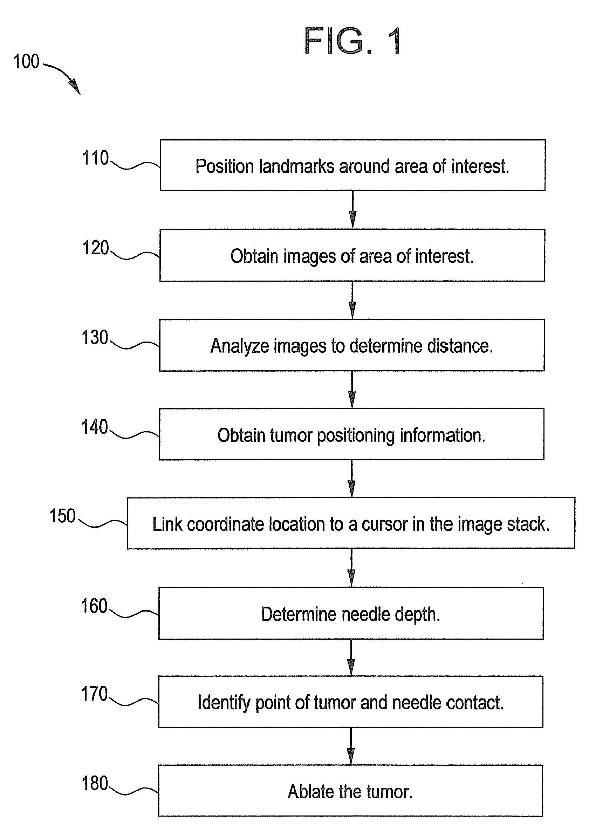

FIG. 1 illustrates a flow diagram for a method 100 for tumor ablation in accordance with an embodiment of the present invention. First, at step 110, a patient is fitted with two or more anatomical landmarks bounding an area of interest. For example, two metal rods may be placed on two sides of an area of interest including a tumor. The landmarks may be placed, taped, affixed, and/or otherwise positioned on or near the patient with respect to the area of interest. The landmarks may be chosen to appear in all axial image slices of the area of interest, for example.

At step 120, images of the area of interest are obtained. For example, a CT exam is performed without a contrast dye injection and with a contrast dye injection. The CT exam series is used to identify the guide landmarks in image slices. Then, at step 130, images are analyzed to determine one or more distance(s) between the tumor and the guide landmarks. The distance is marked or otherwise indicated on the images. Image acquisition and analysis may be performed before or during a tumor ablation procedure, for example.

At step 140, positional information regarding the tumor is obtained during an ablation procedure. For example, a needle electrode is inserted at a tumor site, and a sensor or other tracking device affixed and/or incorporated with a needle electrode or other ablation instrument provides a 2D cursor coordinate (e.g., an X-Y coordinate) on an image, such as an x-ray fluoroscopic image. At step 150, the 2D coordinate location is linked to a cursor in the previously acquired CT exam image stack. A coordinate location or cursor may be displayed on both an image from the CT image stack and an x-ray fluoroscopic image being acquired during the ablation procedure, for example.

At step 160, a depth of the needle electrode is determined using the CT exam image stack showing the needle electrode is shown in the axial plane. At step 170, a point of tumor and needle contact is identified in the CT image stack by locating the starting and ending slices of the tumor in the image stack. Thus, a user, such as an interventional radiologist, is provided with 2D (e.g., x-y plane) coordinates for a tumor as well as 3D (e.g., z plane) depth to more accurately locate and ablate a tumor. Tumor positional information, tumor-to-landmark distance information, and needle depth may be used to produce tumor coordinates, for example. Tumor and needle coordinate may be displayed on both an image slice and a dynamic image to guide a user in an ablation procedure.

At step 180, the tumor is ablated. For example, the needle electrode may be heated to a desired temperature to cook the tumor for a certain period of time. The needle may be advanced to continue to ablate the tumor. Coordinate data may be used to help a user more accurately ablate all or part of a tumor, for example. Coordinate data may provide a user with more accurate information to cook tumor cells.

The steps of the method 100 may be performed in a plurality of orders. In an embodiment, some steps of the method 100 may be eliminated and/or modified. In an embodiment, the method 100 may be applied to an endoscopic procedure as well.

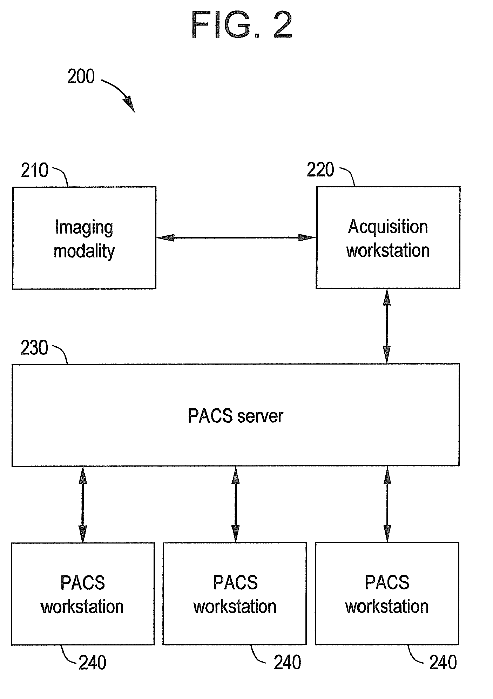

FIG. 2 illustrates an exemplary Picture Archiving and Communication System (PACS) 200 used in accordance with an embodiment of the present invention. The PACS system 200 includes an imaging modality 210, an acquisition workstation 220, a PACS server 230, and one or more PACS workstations 240. The system 200 may include any number of imaging modalities 210, acquisition workstations 220, PACS server 230 and PACS workstations 240 and is not in any way limited to the embodiment of system 200 illustrated in FIG. 2. The components of the system 200 may communicate via wired and/or wireless communication, for example, and may be separate systems and/or integrated to varying degrees, for example.

In operation, the imaging modality 210 obtains one or more images of a patient anatomy. For example, a series or stack of CT image slices may be obtained of a patient anatomy. The imaging modality 210 may include any device capable of capturing an image of a patient anatomy such as a medical diagnostic imaging device. For example, the imaging modality 210 may include an X-ray imager, ultrasound scanner, magnetic resonance imager, or the like. Image data representative of the image(s) is communicated between the imaging modality 210 and the acquisition workstation 220. The image data may be communicated electronically over a wired or wireless connection, for example.

In an embodiment, the acquisition workstation 220 may apply one or more preprocessing functions, for example, to the image data in order to prepare the image for viewing on a PACS workstation 240. For example, the acquisition workstation 220 may convert raw image data into a DICOM standard format or attach a DICOM header. Preprocessing functions may be characterized as modality-specific enhancements, for example (e.g., contrast or frequency compensation functions specific to a particular X-ray imaging device), applied at the beginning of an imaging and display workflow. The preprocessing functions differ from processing functions applied to image data in that the processing functions are not modality specific and are instead applied at the end of the imaging and display workflow (for example, at a display workstation 240).

The image data may then be communicated between the acquisition workstation 220 and the PACS server 230. The image data may be communicated electronically over a wired or wireless connection, for example.

The PACS server 230 may include computer-readable storage media suitable for storing the image data for later retrieval and viewing at a PACS workstation 240, for example. The PACS server 230 may also include one or more software applications for additional processing and/or preprocessing of the image data by one or more PACS workstations 240.

One or more PACS workstations 240 are capable of or configured to communicate with the server 230 and/or other system, for example. The PACS workstations 240 may include a general purpose processing circuit, a PACS server 230 interface, a software memory, and/or an image display monitor, for example. The PACS server 230 interface may be implemented as a network card connecting to a TCP/IP based network, but may also be implemented as a parallel port interface, for example.

The PACS workstations 240 may retrieve or receive image data from the server 230 and/or other system for display to one or more users. For example, a PACS workstation 240 may retrieve or receive image data representative of a computed radiography (CR) image of a patient's chest. A radiologist or user may then examine the image(s) for any objects of interest, such as tumors, lesions, etc., for example.

The PACS workstations 240 may also be capable of or configured to apply processing functions to image data. For example, a user may desire to apply processing functions to enhance features within an image representative of the image data. Processing functions may therefore adjust an image of a patient anatomy in order to ease a user's diagnosis of the image. Such processing functions may include any software-based application that may alter a visual appearance or representation of image data. For example, a processing function can include any one or more of flipping an image, zooming in an image, panning across an image, altering a window and/or level in a grayscale representation of the image data, and altering a contrast and/or brightness an image.

In an embodiment, the PACS system 200 may provide one or more perspectives for viewing images and/or accessing applications at a PACS workstation 240. Perspectives may be provided locally at the PACS workstation 240 and/or remotely from the PACS server 230. In an embodiment, the PACS system 200 includes a perspectives manager capable of being used for reviewing images via a plurality of perspectives. The PACS server 230 and/or a PACS workstation 240 may include the perspectives manager, or the perspectives manager may be implemented in a separate system. In an embodiment, each PACS workstation 240 may include a perspectives manager.

In operation, for example, a user, such as a radiologist, selects a set of images, such as screening mammogram images, chest screening images and/or other computed radiography (CR), digital radiography (DR), and/or digital x-ray (DX) screening images, to review at a PACS workstation 240. The images may be displayed in a default perspective and/or a customized perspective, for example.

As described above, a user may wish to apply additional processing steps to one or more images to further enhance features in the image. For example, a user may desire to apply additional processing functions or steps to an image in order to alter the presentation of an image in conformance with the user's confidence level for making an accurate diagnosis. In other words, different users may desire to apply different or additional processing steps than are included in a default image processing workflow.

The additional image processing step(s) may include any image processing step useful to prepare an image for a diagnostic examination. For example, as described above, an image processing step (as a default image processing step or an additional image processing step) may include flipping an image, zooming in an image, panning across an image, and altering one or more of a window, a level, a brightness and a contrast setting of an image. Image data may be displayed on a PACS workstation 240 using the same and/or different processing, display protocol, and/or perspective as other image(s), for example.

PACS workstations 240 may retrieve or receive image data from server 230 for display to one or more users. For example, a PACS workstation 240 may retrieve or receive image data representative of a computed radiography image of a patient's chest. A radiologist may then examine the image as displayed on a display device for any objects of interest such as, for example, tumors, lesions, etc.

PACS workstations 240 may also be capable of or configured to retrieve and/or receive one or more hanging protocols from server 230. For example, a default hanging protocol may be communicated to PACS workstation 240 from server 230. A hanging protocol may be communicated between server 230 and a PACS workstation 240 over a wired or wireless connection, for example.

In general, PACS workstations 240 may present images representative of image data retrieved and/or received from server 230. PACS workstations 240 may present the images according to a hanging protocol. As described above, a hanging protocol is a set of display rules for presenting, formatting and otherwise organizing images on a display device of a PACS workstation 240. A display rule is a convention for presenting one or more images in a particular temporal and/or spatial layout or sequence. For example, a hanging protocol may include a set of computer-readable instructions (or display rules, for example) that direct a computer to display a plurality of images in certain locations on a display device and/or display the plurality of images in a certain sequence or order. In another example, a hanging protocol may include a set of computer-readable instructions that direct a computer to place a plurality of images in multiple screens and/or viewports on a display device. In general, a hanging protocol may be employed to present a plurality of images for a diagnostic examination of a patient anatomy featured in the images.

A hanging protocol may direct, for example, a PACS workstation 240 to display an anterior-posterior ("AP") image adjacent to a lateral image of the same anatomy. In another example, a hanging protocol may direct PACS workstation 240 to display the AP image before displaying the lateral image. In general, a hanging protocol dictates the spatial and/or temporal presentation of a plurality of images at PACS workstation 240.

A hanging protocol differs from a default display protocol ("DDP"). In general, a DDP is a default workflow that applies a series of image processing functions to image data. The image processing functions are applied to the image data in order to present an image (based on the image data) to a user. The image processing functions alter the appearance of image data. For example, an image processing function may alter the contrast level of an image.

DDPs typically include processing steps or functions that are applied before any diagnostic examination of the images. For example, processing functions may be applied to image data in order to enhance features within an image (based on the image data). Such processing functions can include any software-based application that may alter a visual appearance or representation of image data. For example, a processing function can include any one or more of flipping an image, zooming in an image, panning across an image, altering a window and/or level setting in a representation of the image data, and altering a contrast and/or brightness setting in a representation of the image data.

DDPs are usually based on a type of imaging modality used to obtain the image data. For example, image data obtained with a C-arm imaging device in general or a particular C-arm imaging device may have a same or similar DDP applied to the image data. In general, a DDP attempts to present image data in a manner most useful to many users. Conversely, applying a hanging protocol to image data does not alter the appearance of an image (based on the image data), but instead dictates how the image(s) is (are) presented, as described above.

Server 230 may store a plurality of hanging protocols and/or DDPs. The hanging protocols and/or DDPs that are stored at server 230 and have not yet been modified or customized are default hanging protocols/DDPs. A default hanging protocol and/or DDP may be selected from a plurality of default hanging protocols and/or DDPs based on any number of relevant factors such as, for example, a manual selection, a user identity, and/or pre-processing of the image data.

For example, a default protocol may be selected based on pre-processing of image data. Pre-processing of image data may include any image processing known to those of ordinary skill in the art that prepares an image for review by a user. Pre-processing may also include, for example, a computer-aided diagnosis ("CAD") of image data. CAD of image data may include a computer (or similar operating unit) automatically analyzing image data for objects of interest. For example, a CAD may include a software application that analyzes image data for nodules in images of lungs, lesions, tumors, etc. However, a CAD application may include any automatic analysis of image data known to those of ordinary skill in the art.

PACS users often wish to run multiple applications on a PACS workstation 240. In addition to a primary PACS workflow or interface application, a user may wish to access other applications such as surgical planning tools, scheduling tools, electronic mail viewers, image processing tools, and/or other tools. For example, PACS users often like to use a PACS workflow engine while viewing electronic mail and accessing information on the Internet. Users of an integrated RIS/PACS system may wish to access both RIS and PACS applications simultaneously.

In an embodiment, a user, such as a radiologist, may obtain a series of CT image slices using a CT imager, for example. The images may be stored as a CT image stack at the PACS server 230. Guiding landmarks, such as metal rods and/or other materials visible in an image, may be positioned, taped, laid, and/or otherwise affixed on or in a patient to identify an area of interest, for example, an area including a tumor. In an embodiment, anatomical landmarks found in a patient may be used to identify an area of interest. Images including the tumor and landmarks may be obtained and stored at the PACS server 230 and/or workstation 240, for example.

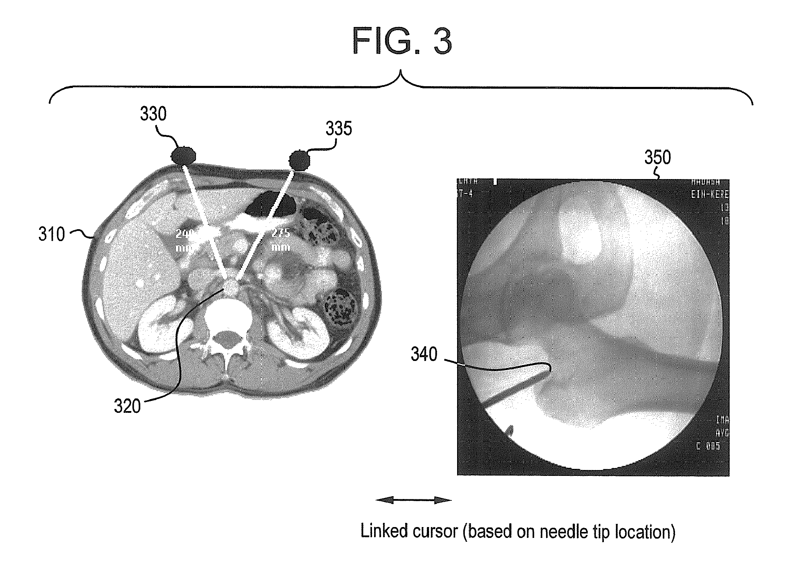

The acquired images may be processed and/or analyzed via a PACS workstation 240, for example. A distance between the tumor and one or more of the landmark guides may be determined from the images, for example. For example, triangulation and/or other distance measurement algorithm may be used to determine the distance. In an embodiment, as illustrated in FIG. 3, the distance(s) between the tumor 320 and the landmarks 330, 335 are marked on the image 310. For example, a depth of the tumor 320 in a patient may be estimated based on one or more cross-sectional images of the patient using the landmarks 330, 335 and tumor 320 locations.

During a tumor ablation procedure, a fluoroscopic image 350 may be obtained to guide a user, such as a surgeon or interventional radiologist. A transmitter, sensor, and/or other tracking device positioned, integrated, and/or otherwise affixed to the needle electrode 340 may transmit positional data. The positional coordinate data may be combined with the fluoroscopic image 350 and/or CT image 310 to show the position of the needle tip 340 with respect to the area of interest. Needle depth may be determined as the needle is inserted into the area of interest. Therefore, a user may dynamically receive positional feedback during tumor ablation to allow the user to more accurate navigate and ablate or cook a tumor. Coordinate information (e.g., X-Y coordinate information) may be obtained from the tip of the needle 340 and linked with one or more previously obtained images 310 (e.g., CT image(s)) and/or fluoroscopic image(s) 350 to indicate tumor 320 start and end locations with respect to the needle tip 340.

For example, a needle electrode 340 may be inserted into a patient in an area of interest including a tumor 320. Positional data regarding the needle 340 location may be combined with image data regarding the tumor 320 site. The needle electrode 340 may be expanded and/or otherwise positioned in the tumor 320 to heat the tumor 320 at a certain temperature. The needle 340 may be maneuvered through the tumor 320 until the tumor 320 is ablated to the user's satisfaction. Tumor 320 and needle 340 location information may be displayed on one or more static and dynamic images simultaneously to assist in accurate and efficient tumor ablation.

Certain embodiments of the system and methods described above may be implemented in software, hardware, and/or firmware, for example. Certain embodiments may provide a computer-readable medium including a set of instructions for execution on a computer or other processor. The set of instructions may include a distance routine configured to determine at least one distance between a tumor and a plurality of landmarks identified in at least one image slice of a patient area, a tracking routine capable of obtaining positional data for an ablation instrument, and a display routine for displaying three-dimensional position information for the ablation instrument with respect to the tumor based on the positional data.

In certain embodiments, the display routine displays a cursor location indicative of the ablation instrument concurrently on the at least one image slice of a patient area and a fluoroscopic image of the patient area. In certain embodiments, the display routine dynamically displays a location of a tip of the ablation instrument with respect to a starting location and an ending location of the tumor. In certain embodiments, the display routine indicates the at least one distance between the tumor and the plurality of landmarks on a dynamically-updating image displayed during tumor ablation. In certain embodiments, the set of instruction may include additional instructions and/or routines in accordance with systems and methods described above.

Thus, certain embodiments combine interventional medical treatment with imaging techniques, such as linking a cursor to positional data. Certain embodiments help reduce time and repetition involved in tumor ablation through more accurate positioning and locational feedback. Certain embodiments provide location information for a tumor and an ablation instrument in a 2D and/or 3D coordinate system.

While the invention has been described with reference to certain embodiments, it will be understood by those skilled in the art that various changes may be made and equivalents may be substituted without departing from the scope of the invention. In addition, many modifications may be made to adapt a particular situation or material to the teachings of the invention without departing from its scope. Therefore, it is intended that the invention not be limited to the particular embodiment disclosed, but that the invention will include all embodiments falling within the scope of the appended claims.

## Claims

1. A method (100) for improved tumor ablation, said method (100) comprising:
determining a distance between a tumor and at least one of a plurality of landmarks bounding said tumor in at least one acquired image of an area of interest for a patient (130);
obtaining positional data for an ablation instrument (140); and
displaying a position of said ablation instrument with respect to said tumor (150).

2. The method (100) of claim 1, wherein said displaying step further comprises dynamically displaying a position of said ablation instrument on at least one of a fluoroscopic image and said at least one acquired image during tumor ablation.

3. The method (100) of claim 2, wherein a location cursor on said fluoroscopic image is linked with a location cursor on said at least one acquired image (150).

4. The method (100) of claim 1, wherein said displaying step further comprises dynamically displaying a location of a tip of said ablation instrument with respect to a starting location and an ending location of said tumor (170).

5. The method (100) of claim 1, wherein said determining step further comprises determining distances between said tumor and said plurality of landmarks to identify a location of said tumor in said at least one acquired image (140).

6. The method (100) of claim 1, further comprising marking said distance between said tumor and at least one of said landmarks on said at least one acquired image.

7. A tumor ablation system, said system comprising:
a processing unit receiving positional data from an ablation instrument (340); and
a display unit displaying a dynamically-updating image (350) of an area of interest and at least one image slice (310) depicting said area of interest, wherein each of said at least one image slice (310) includes a depiction of a tumor (320) and a plurality of landmarks (330, 335) surrounding said tumor (320), and wherein said display unit displays a position of said ablation instrument (340) concurrently on said dynamically-updating image (350) and on said at least one image slice (310).

8. The system of claim 7, wherein said display unit displays said position of said ablation instrument (340) and a position of said tumor (320) in a three-dimensional coordinate system.

9. The system of claim 7, wherein said display unit facilitates indicating a distance between at least one of said plurality of landmarks (330, 335) and said tumor (320) on at least one of said dynamically-updating image (350) and said at least one image slice (310).

10. The system of claim 7, wherein said display unit dynamically displays a tip of said ablation instrument (340) with respect to a starting location and an ending location of said tumor (320).
